Europäisches Patentamt

European Patent Office

Office européen des brevets

(11) Veröffentlichungsnummer: **0 364 852**
**A2**

## (12) EUROPÄISCHE PATENTANMELDUNG

(21) Anmeldenummer: **89118736.1**

(22) Anmeldetag: **09.10.89**

(51) Int. Cl.5: **C07H 15/04**

Patentansprüche für folgenden Vertragsstaat: ES.

(30) Priorität: **15.10.88 DE 3835199**

(43) Veröffentlichungstag der Anmeldung:
**25.04.90 Patentblatt 90/17**

(84) Benannte Vertragsstaaten:
**AT BE CH DE ES FR GB IT LI NL SE**

(71) Anmelder: **BASF Aktiengesellschaft**
**Carl-Bosch-Strasse 38**
**D-6700 Ludwigshafen(DE)**

(72) Erfinder: **Baur, Richard, Dr.**
**Nelkenstrasse 1**
**D-6704 Mutterstadt(DE)**
Erfinder: **Houben, Jochen, Dr.**
**Benzstrasse 93**
**D-6520 Worms 26(DE)**
Erfinder: **Oftring, Alfred, Dr.**
**Im Rouhrich 49**
**D-6702 Bad Duerkheim(DE)**
Erfinder: **Stoeckigt, Dieter**
**Koenigstrasse 4**
**D-6700 Ludwigshafen(DE)**

(54) **Substituierte Glucoside.**

(57) Substituierte Glucoside I

$$(Glu_m-R^1)R_n^2 \qquad (I)$$

in der

Glu eine Glucose-Einheit bezeichnet,

$R^1$ einen $C_8$- bis $C_{18}$-Alkylrest in Acetalbindung bedeutet,

$R^2$ für $C_1$- bis $C_4$-Alkylgruppen oder Arylmethylgruppen in Etherbindung steht,

m einen mittleren Wert von 1 bis 10 und

n einen mittleren Wert von 0,1m bis 2m

hat.

EP 0 364 852 A2

## Substituierte Glucoside

Die vorliegende Erfindung betrifft neue substituierte Glucoside der allgemeinen Formel I

$(Glu_m\text{-}R^1)R_n^2$     I

in der

Glu eine Glucose-Einheit bezeichnet,

$R^1$ einen $C_8$- bis $C_{18}$-Alkylrest in Acetalbindung bedeutet,

$R^2$ für $C_1$- bis $C_4$-Alkylgruppen oder Arylmethylgruppen in Etherbindung steht,

m einen mitleren Wert von 1 bis 10 und

n einen mittleren Wert von 0,1m bis 2m

hat.

Außerdem betrifft die Erfindung ein Verfahren zur Herstellung der Glucoside I und von Gemischen dieser Glucoside sowie ihre Verwendung als oberflächenaktive Wirksubstanzen in Wasch-, Reinigungs- und Körperpflegemitteln.

In der DE-A 19 43 689 (1) werden oberflächenaktive Alkyl-Oligosaccharide der Formel RO $(C_6H_{10}O_5)_rH$ in der der Rest R einen $C_{11}$- bis $C_{32}$-Alkylrest bedeutet und r den Wert von mindestens 2, im Mittel aber 3 oder mehr hat, beschrieben. Die DE-B 19 05 523 (2) und die DE-A 32 32 791 (3) betreffen Verfahren zur Herstellung von Glucosiden oder Glucosid-Gemischen, beispielsweise bei (2) durch Acetalisierung von Glucose mit einem einwertigen $C_8$- bis $C_{25}$-Alkohol in Gegenwart einer Säure; dabei wird in einer bevorzugten Ausführungsform die Glucose zuerst mit einem primären oder sekundären $C_3$- bis $C_5$-Alkohol umgesetzt und dann zum längerkettigen Alkylglucosid umacetalisiert. In (3) wird ein verbessertes Verfahren zur Herstellung von $C_3$- bis $C_5$-Alkylglucosiden in Gegenwart von beispielsweise Natriumperborat beschrieben, das hellere Produkte mit niedrigerem Kondensationsgrad r liefert.

Die in (1) und (2) genannten längerkettigen Alkylglycoside wurden bislang hauptsächlich als Reiniger zur Fettablösung im industriellen Bereich eingesetzt. Einem weitergehenden Einsatz dieser Substanzen als Tenside oder Emulgatoren in Wasch-, Reinigungs- und Körperpflegemitteln stand bisher die zu niedrige Hydrophobie dieser Verbindungen entgegen, welche eine zu geringe Absenkung der Oberflächenspannung und eine zu starke Schaumbildung in den diese Substanzen enthaltenden Zubereitungen bewirkt.

Der Erfindung lag daher die Aufgabe zugrunde, den beschriebenen Mängeln abzuhelfen.

Demgemäß wurden die eingangs definierten substituierten Glucoside I gefunden.

Der Rest $R^1$ bezeichnet einen längerkettigen Alkylrest mit 8 bis 18, vorzugsweise 8 bis 14 C-Atomen, der mit dem 1-C-Atom des Glucosemoleküls acetalisch verknüpft ist. Als Reste $R^1$ kommen zum Beispiel die Octyl-, Nonyl-, iso-Nonyl-, Decyl-, Undecyl-, Lauryl-, Tridecyl-, iso-Tridecyl-, Myristyl-, Cetyl- und Stearylgruppe in Betracht.

$R^2$ steht für $C_1$- bis $C_4$-Alkylgruppen oder Arylmethylgruppen, die mit dem 2-C, 3-C, 4-C und/oder 6-C-Atom des Glucosemoleküls jeweils über einen Ether-Sauerstoff verbunden sind. Als Beispiele für $R^2$ sind vor allem die Methyl-, Ethyl- und Benzylgruppe und daneben die Propyl- und die Butylgruppe zu nennen.

Der Kondensationsgrad m liegt zwischen 1 und 10, vorzugsweise zwischen 1 und 5. Das Auftreten von Werten für m, die größer als 1 sind, ist durch die Herstellung der Vorstufen von I durch Acetalisierung von beispielsweise Glucose im sauren Medium bedingt, bei der zwangsläufig als Nebenreaktion Kondensationsprozesse zwischen den Glucosemolekülen ablaufen. In der Regel liegen Gemische der Glucoside I vor, bei denen m einen Mittelwert darstellt. In diesen Gemischen sind auch in geringen Mengen Glucoside I mit Werten für m von größer als 10 zu finden.

Der Veretherungsgrad n hat einen Wert zwischen 0,1m und 2m, vorzugsweise zwischen 0,1m und m. Es liegen in der Regel Gemische der Glucoside I mit unterschiedlichem Veretherungsgrad n vor, so daß n einen Mittelwert darstellt.

Die erfindungsgemäßen substituierten Glucoside I oder Gemische dieser Glucoside werden vorteilhaft aus den Glucosiden II

$Glu_m\text{-}R^1$     II

durch Umsetzung mit einer oder mehreren Verbindungen III

$R^2\text{-}X$     III

hergestellt, wobei X eine nucleophile Abgangsgruppe bedeutet. Diese Umsetzung erfolgt in an sich bekannter Weise in wäßrig-alkalischer Lösung.

Die als Vorstufe zu I dienenden unsubstituierten Glucoside II und deren Gemische haben in der Regel Kondensationsgrade m von im Mittel 1,5 bis 3.

Pro Mol II werden 0,1m bis 2m mol, vorzugsweise 0,1m bis m mol III eingesetzt. Als Verbindungen III mit nucleophiler Abgangsgruppe kommen Methylchlorid, Ethylchlorid, n-Propylchlorid, iso-Propylchlorid, n-

2

Butylchlorid, iso-Butylchlorid, sec-Butylchlorid, tert-Butylchlorid, Benzylchlorid sowie die entsprechenden Brom- und Jodverbindungen in Betracht, außerdem können Dimethylsulfat, Diethylsulfat, Dipropylsulfat und Dibutylsulfat verwendet werden. Es können auch Gemische dieser Alkylierungsmittel eingesetzt werden. Die Umsetzung von II mit III wird beispielsweise in wäßriger Natronlauge, Kalilauge oder Ammoniaklösung vorgenommen.

Die erfindungsgemäßen Glucoside I finden als oberflächenaktive Wirksubstanzen Verwendung, hauptsächlich als nichtionogene Tenside oder Emulgatoren in Wasch- und Reinigungsmitteln, beispielsweise für Reinigungsprozesse in Technik und Haushalt wie für die Textilwäsche oder für Reinigungsprozesse im Ernährungsmittelbereich wie die Reinigung von Getränkeflaschen. Weiterhin dienen sie in Körperpflegemitteln wie Hautcremes oder in Haarshampoos als Emulgatoren. Diese Wasch-, Reinigungs- und Körperpflegemittel enthalten 20 bis 70 Gew.-%, vorzugsweise 30 bis 50 Gew.-%, bezogen auf die Gesamtmenge der Zubereitung, der erfindungsgemäßen Glucoside I oder von deren Gemischen.

Die erfindungsgemäßen Glucoside I zeichnen sich neben ihren guten Anwendungseigenschaften wie der deutlichen Absenkung der Oberflächenspannung, geringem Schaumvermögen und ausreichendem Netzvermögen durch ihre gute biologische Abbaubarkeit aus. Während die in (1) und (2) genannten längerkettigen Alkylglucoside sich schon beispielsweise während der Lagerung in Gegenwart von Mikroorganismen zu einem merklichen Teil abbauen können, bevor sie als Reinigungsmittel zur Anwendung gelangen, besteht dieser Nachteil bei den erfindungsgemäßen Glucosiden nicht; dagegen werden die Glucoside 1 nach ihrer bestimmungsgemäßen Verwendung schon in den Kläranlagen fast vollständig abgebaut.

Ein weiterer Vorteil ist die hohe Reinheit der nach dem erfindungsgemäßen Verfahren hergestellten Glucoside I, denn nicht umgesetztes III kann durch Erhitzen des Reaktionsgemisches in alkalischer Lösung vollständig zerstört, der dabei entstandene Alkohol durch Destillation leicht entfernt und gleichzeitig entstandenes Salz häufig durch Zugabe von Wasser und Erhitzen über den Trübungspunkt unproblematisch mit der leichteren wäßrigen Phase abgetrennt werden.

Beispiele

Als Ausgangsverbindung und als Vergleichsprodukt wurde ein gemäß (2) und (3) aus Glucose und einem $C_{10}$- bis $C_{12}$-Alkanol-Destillationsschnitt hergestelltes Glucosid II verwendet, dessen Kondensationsgrad m im Mittel bei 2,6 bis 2,8 lag.

Beispiel 1

700 g (0,90 mol) des Glucosids II wurden in 300 g Wasser gelöst und mit 100 g 50 gew.-%iger Natronlauge (entsprechend 1,25 mol NaOH) versetzt, wobei die Temperatur auf 44°C anstieg. Bei dieser Temperatur wurden 101,3 g (0,80 mol) Benzylchlorid innerhalb von 1 Stunde zugetropft. Anschließend wurde die Reaktionsmischung noch 6 Stunden bei 60°C gerührt. Nach Zugabe von 500 g Wasser wurden bei einem Druck von 130 mbar 122 g Flüssigkeit abdestilliert, welche aus Wasser und geringen Mengen Benzylalkohol bestand, die zusammen ein azeotropes Gemisch bilden. Man erhielt 1580 g einer wäßrigen Lösung der substituierten Glucoside 1 mit einem Feststoffgehalt von 46,5 Gew.-%.

Das Produkt hatte einen mittleren Aralkylierungsgrad von n = 0,9 und einen mittleren Kondensationsgrad von m = 2,6 bis 2,8.

Beispiel 2

700 g (0,90 mol) des Glucosids II wurden in 300 g Wasser gelöst und mit 152 g 50 gew.-%iger Natronlauge (entsprechend 1,90 mol NaOH) versetzt, wobei die Temperatur auf 48°C anstieg. Bei dieser Temperatur wurden 152 g (1,20 mol) Benzylchlorid innerhalb von 2 Stunden zugetropft. Anschließend wurde die Reaktionsmischung noch 11 Stunden bei 80°C gerührt. Nach Zugabe von 100 g Wasser wurde zum Sieden erhitzt. Durch azeotrope Destillation wurden 100 g Flüssigkeit abgetrennt, welche aus Wasser und geringen Mengen Benzylalkohol bestand. Bei 90 bis 95°C wurden die Phasen getrennt. Die untere Phase, die die substituierten Glucoside I enthielt, wog 1613 g und hatte einen Feststoffgehalt von 50,3 Gew.-%.

Das Produkt hatte einen mittleren Aralkylierungsgrad von n = 1,3 und einen mittleren Kondensationsgrad von m = 2,6 bis 2,8.

3

Beispiel 3

700 g (0,90 mol) des Glucosids II wurden in 300 g Wasser gelöst und mit 200 g 50 gew.-%iger Natronlauge (entsprechend 2,50 mol NaOH) versetzt, wobei die Temperatur auf 43°C anstieg. Bei dieser Temperatur wurden 203 g (1,60 mol) Benzylchlorid innerhalb von 0,5 Stunden zugetropft. Anschließend wurde die Reaktionsmischung noch 14 Stunden bei 80°C gerührt. Nach Zugabe von 2000 g Wasser wurde zum Sieden erhitzt. Durch azeotrope Destillation wurden 150 g Flüssigkeit abgetrennt, welche aus Wasser und geringen Mengen Benzylalkohol bestand. Bei 90 bis 95°C wurden die Phasen getrennt. Die untere Phase, die die substituierten Glucoside I enthielt, wog 1160 g und hatte einen Feststoffgehalt von 56,5 Gew.-%.

Dieses Produkt hatte einen mittleren Aralkylierungsgrad von n = 1,8 und einen mittleren Kondensationsgrad von m = 2,6 bis 2,8.

Als Anwendungseigenschaften der Glucoside I wurden die Oberflächenspannung, das Schaumvermögen und das Netzvermögen von wäßrigen Zubereitungen, die die erfindungsgemäßen Glucosid-Gemische aus den Beispielen 1 bis 3 enthielten, untersucht. Außerdem wurde der Trübungspunkt als Maß für die Hydrophobie der oberflächenaktiven Substanz gemessen.

Die Oberflächenspannung wurde nach DIN 53914 bestimmt. Dabei wird die Kraft in mN/m gemessen, welche notwendig ist, um einen horizontal aufgehängten Ring oder Bügel aus der Flüssigkeitsoberfläche herauszuziehen.

Das Schaumvermögen wurde nach DIN 53902 durch Messung des Schaumvolumens in ml eine Minute nach Beendigung der Schaumerzeugung bestimmt.

Das Netzvermögen wurde nach DIN 53901 durch Tauchen eines Baumwollgewebes in die zu untersuchende Tensidlösung ermittelt. Dabei wird die Zeit in sec gemessen, nach der das Gewebe seinen durch die eingeschlossene Luft verursachten Auftrieb verliert und zu sinken beginnt. Je kürzer die Zeitspanne, desto besser ist das Netzvermögen.

Der Trübungspunkt wurde nach DIN 53917 gemessen. Die hierfür angegebene Vorschrift bezieht sich ursprünglich nur auf Ethylenoxid-Anlagerungsprodukte, wird aber üblicherweise auch auf andere nichtionogene Tenside angewendet. Als Trübungspunkt wird die kritische untere Entmischungstemperatur in °C, bei der sich zwei Phasen bilden, bei Temperaturerniedrigung einer heißen homogenen Mischung des Tensids mit einer 25 gew.-%gen wäßrigen Butyldiglykol-Lösung verstanden. Je tiefer der Trübungspunkt liegt, desto größer ist die Hydrophobie des Tensids.

Die folgende Tabelle zeigt die Ergebnisse mit dem nicht benzylierten Glucosid zum Vergleich und mit den Glucosid-Gemischen der Beispiele 1 bis 3. Für die Bestimmung der Oberflächenspannung wurde jeweils eine wäßrige Lösung mit 0,1 g wasserfreier Wirksubstanz pro Liter verwendet, das Netzvermögen wurde in einer wäßrigen Lösung mit jeweils 1,0 g wasserfreier Wirksubstanz pro Liter ermittelt.

Tabelle

| | Oberflächenspannung bei 20°C [mN/m] | Schaumvermögen [ml] | Netzvermögen bei 25°C [sec] | Trübungspunkt [°C] |
|---|---|---|---|---|
| Glucosid II (als Vergleich) | 36,0 | 550 | 75 | >100 |
| Beispiel 1 | 31,6 | 210 | 160 | >100 |
| Beispiel 2 | 30,2 | 60 | 270 | 85 |
| Beispiel 3 | 31,2 | 50 | 105 | 63 |

**Ansprüche**

1. Substituierte Glucoside der allgemeinen Formel I

$(Glu_m\text{-}R^1)R_n^2$  I

in der

Glu eine Glucose-Einheit bezeichnet,

$R^1$ einen $C_8$- bis $C_{18}$-Alkylrest in Acetalbindung bedeutet,

$R^2$ für $C_1$- bis C(-Alkylgruppen oder Arylmethylgruppen in Etherbindung steht,

m einen mittleren Wert von 1 bis 10 und

n einen mittleren Wert von 0,1m bis 2m
hat.

2. Substituierte Glucoside der allgemeinen Formel I, in der $R^2$ für einen Methyl-, Ethyl- oder Benzylrest steht.

3. Substituierte Glucoside der allgemeinen Formel I, in der n einen Wert von 0,1m bis m hat.

4. Verfahren zur Herstellung von substituierten Glucosiden I gemäß den Ansprüchen 1 bis 3 oder Gemischen dieser Glucoside, dadurch gekennzeichnet, daß man Glucoside der allgemeinen Formel II $\text{Glu}_m$-$R^1$    II
in an sich bekannter Weise mit 0,1 m bis 2 m mol pro Mol II einer oder mehrerer Verbindungen der allgmeinen Formel III $R^2$-X    III
umsetzt, in der X eine nucleophile Abgangsgruppe bedeutet.

5. Verwendung von substituierten Glucosiden I gemäß den Ansprüchen 1 bis 3 oder Gemischen dieser Glucoside als oberflächenaktive Wirksubstanzen in Wasch-, Reinigungs- und Körperpflegemitteln.

6. Wasch-, Reinigungs- und Körperpflegemittel, enthaltend 20 bis 70 Gew.-%, bezogen auf die Gesamtmenge der Zubereitung, der substituierten Glucoside I gemäß den Ansprüchen 1 bis 3 oder der Gemische dieser Glucoside.

Patentansprüche für folgenden Vertragsstaat: ES

1. Verfahren zur Herstellung von substituierten Glucosiden der allgemeinen Formel I
$(\text{Glu}_m\text{-}R^1)R^2_n$    I
in der
Glu eine Glucose-Einheit bezeichnet,
$R^1$ einen $C_8$- bis $C_{18}$-Alkylrest in Acetalbindung bedeutet,
$R^2$ für $C_1$- bis $C_4$-Alkylgruppen oder Arylmethylgruppen in Etherbindung steht,
m einen mittleren Wert von 1 bis 10 und
n einen mittleren Wert von 0,1m bis 2m hat,
dadurch gekennzeichnet, daß man Glucoside der allgemeinen Formel II
$\text{Glu}_m$-$R^1$    II
in an sich bekannter Weise mit n mol pro Mol II einer oder mehrerer Verbindungen der allgmeinen Formeln III
$R^2$-X    III
umsetzt, in der X eine nucleophile Abgangsgruppe bedeutet.

2. Verfahren nach Anspruch 1, dadurch gekennzeichnet, daß man es auf die Herstellung von substituierten Glucosiden I anwendet, in denen $R^2$ für einen Methyl-, Ethyl- oder Benzylrest steht.

3. Verfahren nach Anspruch 1 oder 2, dadurch gekennzeichnet, daß man es auf die Herstellung von substituierten Glucosiden I anwendet, in denen n einen Wert von 0,1 m bis m hat.

4. Verfahren zur Herstellung von Wasch-, Reinigungs- und Körperpflegemitteln, dadurch gekennzeichnet, daß man 20 bis 70 Gew.-% eines substituierten Glucosids gemäß den Ansprüchen 1 bis 3 oder einer Mischung dieser Glucoside, bezogen auf die Gesamtmenge der Zubereitung, mit den hierbei üblichen Bestandteilen mischt.